# EUROPEAN PATENT APPLICATION

(11) **EP 4 478 371 A1**
(43) Date of publication of application: **18.12.2024**
(21) Application number: 23179226.8
(22) Date of filing: 14.06.2023
(51) Int. Cl.: G16H 10/60, G16H 50/30

(54) **PREDICTING THE RELIABILITY OF A VALUE FOR A PARAMETER OF A CLINICAL SUBJECT**

(71) Applicant: Koninklijke Philips N.V., 5656 AG Eindhoven (NL)
(72) Inventor: HUIJBREGTS, Laurentia Johanna, 5656AG Eindhoven (NL); VAN LIESHOUT, Ron Martinus Laurentius, 5656AG Eindhoven (NL); DANISMAN-TASAR, Mine, 5656AG Eindhoven (NL); BULUT, Murtaza, 5656AG Eindhoven (NL)
(74) Representative: Philips Intellectual Property & Standards

(57) **Abstract**

A mechanism for producing a reliability indicator for a value of a parameter of a clinical subject. The reliability indicator is produced by processing time information, that indicates a length of time since the value was recorded/updated, and event information, that identifies the occurrence of any events that effect the value of the parameter since the value was (last) recorded/updated.

## Description

### FIELD OF THE INVENTION

The present invention relates to the field of medical monitoring, and in particular to the reliability of values produced during medical monitoring.

### BACKGROUND OF THE INVENTION

In the medical field, there is a keen and ongoing interest to perform accurate monitoring of a clinical subject. It is typical for a variety of physiological parameters of the clinical subject to be monitored, e.g., to iteratively record values for the physiological parameters. In some instances, these values are further processed to derive further values or measures, e.g., a hemodynamic stability.

It is important for the value(s) of any physiological parameter to be accurate and to reflect the current status of the clinical subject. This ensures that accurate diagnosis, assessment and/or treatment of the clinical subject will take place, as clinicians rely upon such values in order to make clinical decisions.

It is also common for automated alarms to be generated responsive to a value(s) breaching a particular threshold. A clinician can then respond to the alarm to avoid or mitigate deterioration of the clinical subject. This provides a further example of the importance of accurate and reliable values for the parameter(s).

A wide variety of different physiological parameters are monitored or recorded for a subject. These include those that are (typically) continuously monitored (e.g., heartrate, SpO₂, respiratory rate and so on), as well as those that are not (typically) continuously monitored (e.g., urine output, lactate level, hemoglobin level and so on).

There is therefore a desire and clear need to ensure that values of any monitored parameters are reliable. It would therefore be advantageous, for each parameter, to provide an accurate indicator of whether or not the value produced for said parameter is reliable.

### SUMMARY OF THE INVENTION

The invention is defined by the claims.

According to examples in accordance with an aspect of the invention, there is provided a computer-implemented method for estimating the reliability of a value recorded for a parameter of a clinical subject.

The computer-implemented method comprises: receiving time information that identifies a length of time that has elapsed since the value for the parameter was recorded or updated; receiving event information that identifies the occurrence of any of one or more events that, when discounting any effect of any other event, are clinically expected to affect the value for the parameter after the value for the parameter was recorded or updated; and generating, by processing the time information and the event information, a reliability indicator that indicates the reliability of the value for the parameter.

The present disclosure provides an approach for producing a reliability indicator for a parameter value.

It has been recognized that when a caregiver aims to determine a patient status (e.g. for making a diagnosis or determining a treatment strategy), they will usually take the values of multiple physiological parameters into account. If, since the last measurement, there has been a significant change in a value, then it should not be taken into account as being still active in the caregiver's analysis of the current patient status. In other words, the value has become unreliable.

It has been herein recognized that the length of time that a value for parameter stays reliable is affected by the occurrence of one or more events since the value was recorded. In particular, the occurrence of certain events can significantly change a value for a parameter. The specific type of event that could affect a parameter will, of course, depend upon the nature of the parameter in question.

The present disclosure provides a mechanism for assessing the reliability of a value for a parameter that takes into account this recognition. This reduces a likelihood that one or more inaccurate clinical decisions will be made based on inaccurate values for the parameter(s).

The one or more events may comprise at least one interventional event, wherein each interventional event is an event that occurs as a result of an external object or individual interacting with the clinical subject. An external object or individual is any object or individual that excludes the clinical subject themselves.

This embodiment recognizes that the occurrence of an interventional event, such as a medical treatment, can have a significant impact on the accuracy of a previously recorded value for a parameter of the intervened subject. Thus, the reliability of the value will be significantly affected.

The one or more events may comprise at least one environmental event, wherein each environmental event is an event that occurs in a surrounding environment of the clinical subject.

This embodiment recognizes that the environment in which the clinical subject is positioned will impact one or more parameters of that subject. For instance, a heart rate and sodium concentration is likely to increase, and a urine output rate likely to reduce, with increasing room temperatures. As another example, acute or instantaneous noise levels can negatively impact blood pressure levels in a clinical subject. As yet another example, a heart rate is likely to increase for increasing humidity.

Thus, the reliability of a previously recorded value can be impacted by external environmental conditions. The proposed embodiment takes this into account in assessing the reliability of a value of a parameter.

The one or more events may comprise at least one internal event, wherein each internal event is an event that is performed by the clinical subject. The occurrence of internal events can have a significant impact on other parameters of the clinical subject. An internal event is one that is performed (consciously or subconsciously) by the subject.

In some examples, at least one internal event is a patient-conscious event, being an event that is consciously performed by the clinical subject.

This embodiment recognizes that a clinical subject may themselves perform one or more actions that affect the reliability of previously recorded values. For instance, if a clinical subject performs exercise, then the reliability of a lactate concentration is expected to significantly change, affecting the accuracy of a previously recorded value.

A patient-conscious event may be an event that directly affects a parameter (e.g., holding the breath) or indirectly affects a parameter, e.g., eating a meal, performing exercise and so on.

In some examples, at least one internal event is a patient-subconscious event, being an event that is subconsciously performed by the clinical subject.

This embodiment recognizes that actions that are not consciously performed by the clinical subject would affect the reliability of a previously recorded value. For instance, a sleep apnea event may significantly affect the reliability of an SpO2 reading. A patient-subconscious event may be a medical event, such as the onset of bleeding, the occurrence of an infection and so on.

In some embodiments, the method further comprises receiving patient history information, wherein the step of generating the reliability indicator comprises processing the time information, the event information and the patient history information.

This embodiment recognizes that historic events, diagnoses, illnesses, pathologies and so on will impact the length of time that a value for a parameter will remain reliable. For instance, if the subject has been previously diagnosed with sleep apnea, than a recording of an SpO2 level will be reliable for a shorter period of time. Similarly, a blood sugar level will be reliable for shorter period of time if a subject has diabetes, compared to if the subject did not have diabetes.

In some examples, each parameter is associated with a notification threshold, being a threshold which, if breached by the value of the parameter, causes a user-perceptible notification to be generated; and the step of generating the reliability indicator comprises processing the time information, the event information and a determined difference between the value of the parameter and the notification threshold.

This embodiment recognizes the need for increased reliability if the value is near the threshold (to reduce a likelihood that the actual value will breach the threshold whilst the latest recorded value has not).

In some examples, the step of generating the reliability indicator comprises: looking up a time threshold in a look-up table using at least the event information; and controlling the reliability indicator to indicate that the value for the parameter is unreliable responsive to the length of time, identified in the time information, breaching the time threshold.

This provides a highly resource efficient approach for determining a reliability of the value of the parameter. In particular, this technique allows a wide variety of different events to be taken into account in determining a threshold for the time since the value was last recorded/updated.

The step of generating the reliability indicator comprises algorithmically processing the time information and the event information to produce the reliability indicator. This approach allows for additional nuance in determining the reliability of the value of the parameter.

In some examples, the value for the parameter is derived from at least one value for one or more physiological parameters of the clinical subject.

In at least one example, the value for the parameter is a value for a physiological parameter of the clinical subject. Embodiments are particularly advantageous when the parameter is a parameter derived or comprising a non-continuous measurement.

Examples of non-continuous measurement parameters include: parameters from intermittent measurements with advanced hemodynamic monitoring devices such as Pulse Contour Cardiac Output (PiCCO) and pulmonary artery catheter (PAC); parameters from hemodynamic Point-of-Care Ultrasound (POCUS) measurements; non-invasive blood pressure (NIBP) measured with an arm cuff; spot-check measurements such as temperature or oxygen saturation; concentration of a substance in a bodily fluid (such as blood, serum, plasma, sweat, interstitial fluid, urine, saliva, tears and/or exhaled breath); and/or output measurements (such as drainage or urine output rate).

In some examples, time information identifies a length of time between a point in time at which the value for the parameter was recorded or updated and a non-current point in time. This approach allows for historic investigation of the reliability of previous values of the parameter. This can allow, for instance, for more nuanced investigation of previous clinical decisions (e.g., to determine their appropriateness or accuracy) which can influence future or ongoing clinical decisions for that subject.

The method may further comprise controlling a user interface to provide a visual representation of the reliability indicator. This provides a clinician or other user with useful information for making a clinical decision. In particular, the provided information facilitates improved performance of a technical task, namely making a clinical decision (such as a diagnosis, treatment or analysis) by the viewer of the visual representation.

There is also provided a computer program product comprising computer program code means which, when executed on a computing device having a processing system, cause the processing system to perform all of the steps of any herein disclosed method.

There is also provided a processing system for determining the reliability of a value recorded for a parameter of a clinical subject.

The processing system is configured to: receive time information that identifies a length of time that has elapsed since the value for the parameter was recorded or updated; receive event information that identifies the occurrence of any of one or more events that, when discounting any effect of any other event, are clinically expected to affect the value for the parameter after the value for the parameter was recorded or updated; and generate, by processing the time information and the event information, a reliability indicator that indicates a reliability of the value for the parameter.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a subject-monitoring system;
Fig. 2 is a flowchart illustrating a proposed approach;
Fig. 3 illustrates timelines representing occurrences of measurements and events over time;
Fig. 4 is a flowchart illustrating another proposed approach; and
Fig. 5 is a flowchart illustrating yet another proposed approach.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for producing a reliability indicator for a value of a parameter of a clinical subject. The reliability indicator is produced by processing time information, that indicates a length of time since the value was recorded/updated, and event information, that identifies the occurrence of any events that effect the value of the parameter since the value was (last) recorded/updated.

Embodiments are based on the realization that the reliability of a parameter value over time is heavily influenced by the occurrence of events that influence the parameter. By taking such events into account when calculating or otherwise determining the reliability of the value, a more accurate reliability indicator can be determined.

Proposed approaches are particularly advantageous when employed in clinical settings, such as hospitals, clinics, emergency rooms, walk-in centers and so on. However, this is not essential and embodiments could be employed in alternative environments, e.g., domestic settings.

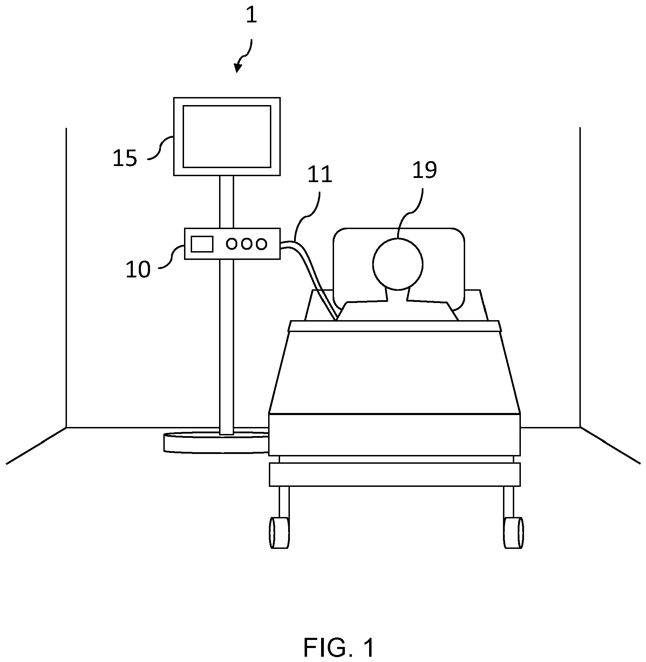

Fig. 1 illustrates a subject-monitoring system 1 in which embodiments of the invention can be employed. The subject-monitoring system 1 comprises a subject-monitoring device 10 and an output generator 15.

Of course, the subject-monitoring device may comprise more than one subject-monitoring module, each adapted to monitor one or more physiological parameters of the subject.

The physiological parameter may be a continuously monitored physiological parameter. Accordingly, the subject-monitoring device 10 may be adapted to monitor one or more physiological parameters of a subject 19. This may be performed, for example, using one or more physiological parameter monitors (not shown) coupled to the subject-monitoring device via cables 11 or wirelessly. Examples of suitable physiological parameter monitors include a pulse oximeter (for measuring SpO2 level and/or heartrate), a heart rate monitor (for measuring heart rate), a blood pressure monitor (for measuring blood pressure) etc.

Examples of physiological parameters include heart rate, mean arterial pressure (MAP), central venous pressure (CVP), cardiac output, respiratory rate, and SpO2 level.

The subject-monitoring device 10 may have access to values for one or more other physiological parameters, e.g., physiological parameters that are not continuously monitored. Examples include urine output, lactate level, hemoglobin level and so on. Other examples include those produced from intermittent measurements with advanced hemodynamic monitoring devices such as Pulse Contour Cardiac Output (PiCCO) and pulmonary artery catheter (PAC / "Swan-Ganz"). Yet other examples include: parameters from hemodynamic Point-of-Care Ultrasound (POCUS) measurements; non-invasive blood pressure (NIBP) (e.g., measured with an arm cuff); temperature, oxygen saturation or other parameters determined from spot-check measurements performed intermittently (e.g. once per shift); the concentration of a substance in a bodily fluid (e.g., blood, serum, plasma, sweat, interstitial fluid, urine, saliva and/or tears) or in exhaled breath (e.g., moisture content); drainage and/or urine output rate. A wide variety of other examples will be apparent to the skilled person.

The subject-monitoring device 10 (or another processing system) may be configured to perform further processing of the value(s) of the monitor physiological parameter(s) to produce a further parameter or measure, such as a hemodynamic stability or the like. Thus, it is possible to derive or produce one or more further parameter(s) from one or more monitored physiological parameters.

The subject-monitoring device 10 may be configured to generate one or more notifications or alarms responsive to the value(s) of the parameter(s) of the subject. Typically, generation of a user-perceptible notification or alarm is responsive to a value of a parameter breaching some predetermined threshold (i.e., an alarm threshold) or the combination of values of different parameters meeting some other predetermined criterion/criteria. Parameters that control or influence the generation of a notification or alarm can be labelled notification parameters or alarm parameters.

A notification or alarm may indicate, for example, the occurrence of a clinically undesirable level of a parameter.

Examples of notifications include notifications indicating: low heart rate; high heart rate; arrhythmia; asystole; low breathing rate; high breathing rate; apnea; low SpO2; high SpO2; low temperature; high temperature; low systolic pressure; high systolic pressure; low diastolic pressure; high diastolic pressure; low mean blood pressure; high mean blood pressure; low perfusion; poor pallor (subject color); too little or too much subject movement; out of bed detection; low/high cardiac output; low/high stroke volume; low/high blood flow; low HSI; high septic risk and so on. Various other notifications or alarms would be apparent to the skilled person, and could be integrated into the inventive concept.

The output generator 15 may be adapted to obtain the notifications or alarms generated by the subject-monitoring device 10. The output generator 15 may communicate with the subject-monitoring device wirelessly or via one or more wires. Notifications may be communicated using respective alarm signals from the subject-monitoring device 10 to the output generator.

The output generator 15 may be configured to generate or produce one or more user-perceptible (i.e., clinician-perceptible) outputs responsive to the alarm. Generating a user-perceptible output may comprise generating an output signal that causes an output device (not shown) to provide a visual, audio and/or haptic output to a clinician.

However, other uses for a notification or alarm will be apparent to the skilled person, e.g., to trigger the automated performance of a further algorithm or clinical intervention.

Regardless of whether or not an alarm is generated for a parameter of the subject 19, it is advantageous if the reliability of the parameter(s) of the subject is known or predicted. This can allow the reliability to be taken into account for any clinical decision process or for the production of any further parameter(s) derived from any such parameter(s) for which a reliability is derived.

Proposed approaches provide techniques for predicting or estimating the reliability of a value recorded for a parameter of the clinical subject and advantageous procedures that make use such a predicted reliability. Embodiments can therefore reduce or avoid error or bias in clinical decision making by a clinician (or algorithmic process) that makes use of any values for the parameter(s) of the clinical subject.

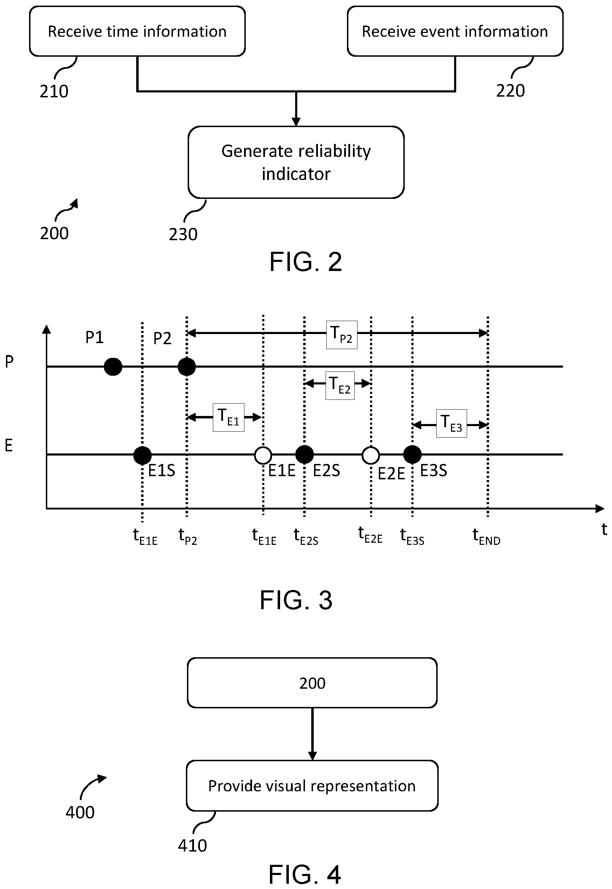

Fig. 2 is a flowchart illustrating a generic computer-implemented method 200 according to the proposed concept. The method 200 is configured for estimating the reliability of a value recorded for a parameter of a clinical subject.

The computer-implemented method 200 comprises a step 210 of receiving time information. The time information identifies a length of time that has elapsed since the value for the parameter was recorded or updated.

The time information may, for instance, identify a length of time between a time at which the parameter was recorded or updated and an end point in time. The end point in time may, for instance, be a current point in time. In other examples, the time information identifies a length of time between the time at which the parameter was recorded/updated and a non-current point in time (e.g., a past point in time or a future point in time). This can allow, for instance, a clinician or algorithmic process to facilitate determination of past reliability indicators for the value for the parameter.

The time information may, for instance, be in the form of a timestamp for the value of the parameter. As another example, the time information may be a measure of the length of time that has elapsed, e.g., which may be expressed numerically, categorically and/or semantically.

The method 200 also comprise a step 220 of receiving event information. The event information identifies the occurrence of any of one or more events. Each event, when discounting any effect of any other event, is clinically expected to affect the value for the parameter after the value for the parameter was recorded or updated.

The event information thereby identifies whether any events will take effect between the last update of the parameter and an end point in time. The event information may, for instance, identify the type of event and/or the number of events. In some examples, the event information identifies the length of time between the event (or the last update of the parameter if later) and the end point in time (of the time information).

The one or more event(s) may be from a restricted list or type of potential event, examples of which will be provided later in this disclosure. In particular, each event in the restricted list effectively represents an events that is excepted to change or modify the value(s) of the parameter at the end point in time.

The event information may further indicate, where relevant, a length of any events. In particular, it is recognized that some events (e.g., a bleeding or performing exercise) may only occur for a finite period of time, and will therefore only affect reliability of a parameter during this finite period of time. The indicated lengths may be used to improve the calculation or determination of the reliability indicator.

The method 200 also comprise a step 230 of generating, by processing the time information and the event information, a reliability indicator that indicates of the value for the parameter.

The reliability indicator may be binary (e.g., an indication of whether or not the value for the parameter is reliable), categorical (e.g., a determination of whether a reliability is "High", "Medium" or "Low") or numeric (e.g., a value or measure of reliability that falls within a predetermined range, e.g. 0-10). It may also indicate the values between which the parameter is expected to lie or the size of this potential range.

It has been herein recognized that the reliability of a parameter for a clinical subject is dependent upon both the length of time that has elapsed since the parameter was last updated and the occurrence of any parameter-influencing events that occur since that same last update. In particular, it has been identified that certain events are likely to influence the value of a parameter, such that a previously recorded value for a parameter is less likely to accurately reflect the true value of the parameter if one or more of these events has occurred.

Specific embodiments proposed various techniques for exploiting this recognition, but the skilled person would be readily capable of performing any other technique that makes use of this innovative recognition.

Fig. 3 is a timeline conceptually illustrating the occurrence of recorded values P1, P2 for a parameter P and the occurrence (or predicted occurrence) of events E1, E2, E3 for one or more events E. Events E1, E2 and E3 are illustrated as having at least a start time E1S, E2S and E3S. Some of the events are illustrated as having an end time E1E, E2E.

The time information identifies a length of time T_{P2} that has elapsed since the value for the parameter was recorded or updated. The length of time T_{P2} may therefore represent a time T_{P2} between the time t_{P2} of the last update P2 for the value of the parameter P and an end point in time t_{END}. As previously mentioned, the end point t_{END} may be a current (or most recently available) point in time or a past/future point in time.

The event information identifies the occurrence of any one or more events E1, E2, E3 that have, or are predicted to have, an effect on the parameter (ignoring the effect of any other events) after the time t_{P2} value for the parameter was last updated. In particular, the event information may identify the occurrence of any events that has an effect that falls between the time t_{P2} at which the parameter was last updated and the end point in time t_{END}, i.e., any events that have an effect that occurs during the period of time T_{P2}.

The event information may, for instance, identify each event E2S, E3S that occurs, e.g., identifies the type of and/or label for the event.

The event information may identify the number of events that occur.

The event information may identify, for each event E2, E3, the length of time T_{E2}, T_{E3} between the time t_{E2}, t_{E3} at which the event occurs (or the time at which the value of the parameter was last updated if later) and the end point in time t_{END}.

In advanced embodiments, the event information may identify, for each event E1, E2, E3, the length of time between the time t_{E2}, t_{E3}, at which the event occurs (or the time at which the value of the parameter was last updated if later) and the time at which the event ends or no longer has an effect on the parameter. For instance, the event information may identify, for each event, the start time of the event and the end time of the event.

Thus, for the example illustrated by Fig. 3, the event information may identify: the length of time T_{E1} that a first event is active since the value of the parameter was last updated; the total length T_{E2} of a second event and the length of time T_{E3} that the third event is active (e.g., until the end point in time).

Turning back to Fig. 2, it has been previously mentioned how each event may be a particular type of event. The relevant event(s) may, for instance, be dependent upon the particular parameter - as different events will affect different parameters to different extents. The relevant event(s) may, for, also/otherwise be dependent upon the subject, as different events will affect (the same parameters of) different subjects to different extents.

In some examples, the one or more events may comprise at least one interventional event. An interventional event is an event that occurs as a result of an external object or individual interacting with the clinical subject. This may include, for instance, one or more clinical intervention events that represent the occurrence of a clinical treatment or procedure (e.g., investigative procedure) performed on the clinical subject. Examples include the provision of medication, saline solutions, application of radiation, application of chemotherapy or other drugs, performance of an imaging technique (e.g., MRI, ultrasound and so on). Other examples include the performance of a treatment by the clinical subject following a recommendation at a user interface, e.g., performing a breathing exercise following a guided intervention at a user interface.

Where relevant, the event information may comprise information pertaining to the event. For instance, if the event is the administration of a particular medication, then the event information may identify the type of medication, the amount of medication and so on. Other suitable examples will be apparent to the skilled person.

The event information may be personalized according to the clinical subject to which it applies. For example, medication effects can be estimated for a specific medical subject (instead of using general baseline values) and these can be made part of the event information.

In some examples, the one or more events comprises at least one environmental event. An environmental event is an event that occurs in a surrounding environment of the clinical subject. Examples include events regarding a temperature (e.g., a temperature beyond some temperature threshold), a moisture level, a CO₂ level, the presence of airborne particles or diseases, the occurrence of a disease in another (nearby) subject, a noise level, a light level, a presence and/or level of natural light and so on.

Where relevant, the event information may comprise information pertaining to the event. For instance, if the event is a temperature, then the event information may carry information about the recorded temperature.

In some examples, the one or more events comprises at least one internal event. An internal event is an event that is performed by the clinical subject (either consciously or sub-consciously).

In particular, at least one internal event may be a patient-conscious event, being an event that is consciously performed by the clinical subject. A conscious event is one that is performed responsive to a decision by the clinical subject, e.g., undergoing exercise, holding their breath, moving position, and so on. The decision by the clinical subject may be made responsive to an external trigger, e.g., a recommendation to perform exercise or instructions on how to perform an exercise. A wide variety of different potential patient-conscious events that are expected to cause a change in a parameter of a clinical subject would be apparent to the skilled person, and may be parameter-dependent.

At least one internal event may be a patient-subconscious event, being an event that is subconsciously performed by the clinical subject. A patient-subconscious event may include any medically relevant event, such as the occurrence of a bleed, elevated heart rate, occurrence of a swelling or the like. Examples of such events include bleeds or changes in one or more other parameters of the clinical subject. For instance, a change in heart rate may be expected to result in a change in a blood pressure. As another example, a change in respiratory rate is expected to result in a change in SpO2. As yet another example, a bleeding is expected to result in a change in haemoglobin level.

Thus, an internal event may be the occurrence of value for another parameter of the clinical subject breaching some threshold and/or one or more other parameters meeting one or more predetermined criteria.

Example approaches for performing step 230 are hereafter described. However, the skilled person would readily appreciate a wide variety of other techniques that could be employed upon being taught that a reliability indicator can be improved by making use of event information as proposed in the present disclosure. The following embodiments are therefore considered to be non-exhaustive and merely exemplary of the underlying inventive recognition of the present disclosure.

One approach to performing step 230 is to make use of a look-up table. In particular, a look-up table may correlate or map (for the parameter) information contained in event information with a time threshold. Responsive to the elapsed time (indicated in the time information) breaching this threshold, an estimation or prediction can be made as to whether the value for that parameter is reliable or not. This provides a mechanism for producing a binary reliability indicator of the most recently recorded/updated value for the parameter.

The look-up table may be located at a same location as the device carrying out the method 200 or may be located externally, e.g., hosted by a web server reachable over the internet or the like.

A time threshold may, for instance, represent a time at which the value of the parameter can no longer be expected (e.g., with a high level of probability) to remain within a particular range with respect to its most recently recorded value, i.e., close to its measured value, e.g., can be expected to have changed by more than 10% (assuming normal or expected bodily function). Thus, the time threshold may effectively represent a time at which the value of the parameter is expected to no longer be sufficiently accurate for the purposes of relying upon it to make a clinical decision (i.e., is no longer reliable).

As later explained, this particular range may be defined by a notification threshold for the value of the parameter. In particular, the particular range may represent a range which, if the value of the parameter is outside, a notification or alarm would be triggered.

As an example, a look-up table may contain a plurality of columns. A first column may identify different parameters. A second (optional) column may identify a default time threshold (for each parameter). The next N columns (the "N event columns") may identify, for N possible events, the time threshold for each of the N events (for each parameter). It is not essential that, for each possible event, a time threshold is provided for each parameter (as some possible events may have no/negligible influence on the value of the parameter).

In this scenario, step 230 may comprise: identifying the parameter in the first column; processing the event information to identify whether any of the N possible events are associated with the parameter (e.g., have an associated time threshold); identifying all time thresholds for the parameter that lie in a same column as any identified possible event associated with the parameter; identifying the smallest time threshold and then determining whether the elapsed time (from the time information) is greater than the time threshold.

Of course, if no event applies to the parameter, then the default time threshold under the default column can/should be used (if available).

In some embodiments, the look-up table may comprise M additional columns indicating a time threshold for a combination of events. Thus, each additional column may represent a different combination of events (e.g., "Bleeding" and "Fluids") that, if present in the event information, are used to identify the corresponding threshold.

**TABLE 1**

| Parameter | Default | Event Bleeding | Fluids | ... | Exercise |
|---|---|---|---|---|---|
| Hemoglobin | 24 hours | 15 minutes | 5 minutes | ... | --- |
| Glucose | 1 hour | --- | 20 minutes | ... | 15 minutes |
| | ... | ... | ... | | |
| Lactate | 3 hours | --- | | ... | 5 minutes |

Table 1 provides a portion of a working example of a suitable look-up table. This exemplifies how for different parameters, different time thresholds can be associated with the occurrence of different events. It is appreciated that a wide variety of different events may be used in practice, and the illustrated example is merely for the purposes of explanative understanding.

The look-up table may, instead of a time threshold being defined as the time at which a maximum of 10% change in the value can be expected, contain another kind of possible rate of change indication. For example, the time threshold may indicate the time at which a maximum of x % change in the value can be expected, where x can be 5, 20, 50 or any other value (preferably with a maximum of 100). As another example, the time threshold may represent the time at which a maximum of y change in the value can be expected, where y is not a percentage, but an absolute amount.

In some examples, the time threshold may be dependent upon a notification or alarm threshold for the parameter. In particular, the time threshold may be equal to a length of time required for the value of the parameter to change as a result of the time passing and, if present, the event occurring, that would result in the triggering of a notification or alarm. This may, for instance, be a length of time that would cause the parameter to breach a predetermined notification threshold and/or one meet one or more other criteria that would cause a notification to be generated. In this way, the reliability indicator may indicate a prediction of whether or not the notification will be triggered if the value of the parameter is updated.

In this way, each parameter may be associated with a notification threshold, being a threshold which, if breached by the value of the parameter, causes a user-perceptible notification to be generated. Generating the reliability indicator may thereby comprise processing the time information, the event information and a determined difference between the value of the parameter and the notification threshold. This approach allows the reliability indicator to indicator whether or not there is a likelihood (e.g., beyond a particular threshold) that the indication threshold will be breached.

Instead of a time threshold, the look-up table may instead contain a speed/rate of change. A rate of change may, for instance, represent an average or maximum expected rate of change in a value of a parameter in a particular scenario (e.g., in the scenario that the corresponding event(s) occur(s)). Thus, the N event columns may instead identify a percentage (%) speed/rate of change (e.g., in % per hr or % per min) of the value of the parameter.

**TABLE 2**

| Parameter | Default | Event Bleeding | Fluids | ... | Exercise |
|---|---|---|---|---|---|
| Hemoglobin | 24 hours | 6.66 % per min | 20 % per min | ... | --- |
| Glucose | 1 hour | --- | 5 % per min | ... | 6.66 % per min |
| | ... | ... | ... | | ... |
| Lactate | 3 hours | --- | | ... | 20 % per min |

Table 2 provides a portion of a working example of a suitable look-up table. This exemplifies how for different parameters, different rates of change (e.g., % per minute) can be associated with the occurrence of different events. It is appreciated that a wide variety of different events may be used in practice, and the illustrated example is merely for the purposes of explanative understanding.

In this scenario, step 230 may comprise: identifying the parameter in the first column; processing the event information to identify whether any of the N possible events are associated with the parameter (e.g., have an associated rate of change); identifying all speeds of change for the parameter that lie in a same column as any identified possible event associated with the parameter; identifying the largest rate of change; processing the elapsed time and the largest rate of change to determine a total expected change (after the elapsed time) and determining whether the total expected change exceeds a predetermined change threshold.

The change threshold may, for instance, represent a change that the value of the parameter can no longer be expected to remain within a particular range with respect to the last recorded value for the parameter, e.g., a change threshold of around 10% or 20%. This particular range may, for instance, be defined by a notification threshold for the value of the parameter. In particular, the particular range may represent a range which, if the value of the parameter is outside, a notification or alarm would be triggered.

The change threshold may be different for different parameters and/or scenarios. In particular examples, parameters monitored using certain monitoring devices may have a lower change threshold then parameters monitored using more reliable monitoring devices. For instance, a parameter of blood pressure monitored using a non-invasive technique may have a lower change threshold (e.g., 10%) than a parameter of pressure monitored using an arterial line.

In some variants, a parameter-dependent value may be added to the total expected change before it is compared to the predetermined change threshold. This approach recognizes that some parameters may be intrinsically more reliable than others. As an example, a parameter of blood pressure monitored using a non-invasive technique may have a higher parameter-dependent value (e.g., 10%) than a parameter of blood pressure monitored using an arterial line (e.g., 0%).

In an example, the change threshold is defined by a notification or alarm threshold for the parameter. In particular, the change threshold may be equal to a percentage change to the (most recent update to) value of the parameter that would result in the triggering of a notification or alarm. This may, for instance, be a percentage change to the value of the parameter that would cause the parameter to breach a predetermined notification/alarm threshold and/or one meeting one or more other criteria that would cause a notification/alarm to be generated.

In this way, the reliability indicator may effectively be a prediction of whether or not the notification/alarm will be triggered if the value of the parameter is updated.

In another example of this scenario, step 230 may comprise: identifying the parameter in the first column; processing the event information to identify whether any of the N possible events are associated with the parameter (e.g., have an associated rate of change); identifying all speeds of change for the parameter that lie in a same column as any identified possible event associated with the parameter; identifying the largest rate of change; processing the elapsed time and the largest rate of change to determine a total expected change (after the elapsed time); subtracting the total expected change from a predetermined value for the parameter and determining whether the result of the subtraction falls below a predetermined percentage threshold.

The predetermined value for the parameter may, for instance, be parameter dependent. In particular, different parameters (e.g., parameters monitored by different monitoring devices) may have different levels of baseline reliability. As an example, a parameter of blood pressure monitored using a non-invasive technique may have a lower predetermined value (e.g., 90%) than a parameter of blood pressure monitored using an arterial line (e.g., 100%).

In some examples, the predetermined percentage threshold is defined by a notification or alarm threshold for the parameter. In particular, the change threshold may be equal to a percentage of the (most recent update to) value of the parameter that would result in the triggering of a notification or alarm. This may, for instance, be a percentage of the value of the parameter that would cause the parameter to breach a predetermined notification/alarm threshold and/or one meeting one or more other criteria that would cause a notification/alarm to be generated.

In this way, the reliability indicator may effectively be a prediction of whether or not the notification/alarm will be triggered if the value of the parameter is updated.

In above-described examples, a single time threshold or a single rate of change is used to determine the reliability indicator. In more advanced examples, multiple time thresholds or rates of change are used. In such circumstances, the event information further identifies, for each event, the length of time that the event is or takes effect within the period since the value was last recorded. In particular, the event information may further indicate, where relevant, a length of each event (within the period since the value was last recorded). As a simple example, the length of time may indicate the length of time that has elapsed since the time at which the event occurs (e.g., between this time and the end point in time).

Reference is made to example elements illustrated in Fig. 3 for the sake of improved contextual understanding.

When using multiple time thresholds or rates of change, a time threshold or rate of change may be determined or calculated for each of one or more periods of time. Each period of time may represent a period, since the last update to the parameter, during which a particular combination of events (or no events) have effect. Neighboring periods of time have different combinations of events. Thus, if X events occur (i.e., are started) since the value was last recorded/updated, then there are at least X+1 periods of time. The number of the periods of time may be equal to the sum of the number of events that start and the number of events that end since the value was last updated. For instance, for the example illustrated in Fig. 3, there are five periods of time (t_{P2} - t_{E1E}; t_{E1E} - t_{E2S}; t_{E2S} - t_{E2E}; t_{E2E} - t_{E3S}; and t_{E3S} -t_{END}).

Where time thresholds are used, the time threshold for each period of time may be calculated or identified using a previously described approach that identifies the shortest time threshold amongst any events that is active or takes effect during the time period. A weighted sum may be performed to calculate a single time threshold against which the time T_{P2} that has elapsed since last update is compared. In particular, the time threshold for each time period may be weighted by the ratio between the associated time period and the (total) elapsed time since the value for the last parameter was updated.

Where rates of change are used, a rate of change for each period of time may be calculated or identified using a previously described approach that identifies the largest rate of change amongst any event (or combination of events) within the combination of events in that period of time. For each period of time, a partial expected change can then be calculated by multiplying the rate of change with the length of the period of time. A total expected change can then be calculated by summing all partial expected changes. The total expected change can then be employed using any previously described approach.

The above-described techniques provide a mechanism for producing a binary reliability indicator. However, the skilled person would be readily capable of adapting such techniques to provide non-binary indicators of reliability.

As a working example, consider a scenario in which the look-up table is used to identify a rate of change for the parameter (based on the relevant event information). Initially, a reliability may be set to a predetermined value (e.g., 100%). Based on the time information and the identified rate of change (e.g., in %/hr), a percentage change of the value can be determined. For instance, if a rate of change is determined to be 10% / hour, and the time information indicates that 1 hour has elapsed since the last update to the value, then the percentage change will be 10%. This can then be subtracted from the predetermined value to establish the reliability of the value - e.g., in a continuation of this example, to 90%.

The predetermined value for the reliability may, for instance, be parameter dependent. In particular, different parameters (e.g., parameters monitored by different monitoring devices) may have different levels of baseline reliability. As an example, a parameter of blood pressure monitored using a non-invasive technique may have a lower predetermined value (e.g., 90%) than a parameter of blood pressure monitored using an arterial line (e.g., 100%).

As yet another example, the reliability indicator may simply be an identification of predicted change (e.g., in terms of percentage) in the value of the parameter. For instance, the percentage change of the value can be determined using an identified rate of change and the time information to determine an expected total change (e.g., in terms of percentage). This can represent the reliability indicator, as the greater this value, the less reliable the value of the parameter. This approach can be readily adapted to use with multiple rates of change, e.g., to calculate the total change in different periods before/between/after the occurrence of one or more events (as indicated in the event information) to more accurate calculate the total expected change.

As another working example, consider another scenario in which the look-up table identifies a time threshold. The reliability indicator may change responsive to a ratio between the elapsed time (since last update of the value) and the time threshold. In particular, an initial numeric value for the reliability indicator may reduce (identifying reducing reliability) as the elapsed time approaches the time threshold. For example, if the time threshold is a value of "15 minutes", then the value of the reliability indicator may decrease (e.g., proportionally) as the elapsed time (since last update of the value for the parameter) approaches the time threshold.

Other suitable examples will be apparent to the skilled person.

In some examples, each event column (and, if present, each additional event column) may be sub-divided into a plurality of event sub-columns. Each event sub-column may identify a time threshold (or rate of change) for different subject categories. A subject category may, for instance, indicate one or more historic events, pathologies, clinical interventions and so on. This information can be used to more accurately identify a reliability indicator for the value of the parameter.

In such approaches, step 230 therefore makes further use of patient history information to produce the reliability indicator.

Thus, step 230 may comprise processing the time information, the event information and the patient history information to produce the reliability indicator.

An alternative to using a look-up table is the use of one or more machine-learning methods. In particular, the time information and the event information may be processed using a suitably trained machine learning method in order to produce a reliability indicator.

In some examples, the time information and the event information is directly processed (by a machine-learning algorithm) to produce the reliability indicator for the parameter.

In alternative examples, the event information is processed by a machine-learning algorithm to produce a time threshold (for the event information). The time information can be used to determine whether or not the time threshold is breached, thereby producing the reliability indicator (e.g., breach = unreliable, no breach = reliable).

In yet other examples, the event information is processed by a machine-learning algorithm to produce or a rate of change (e.g., %/hour) for the parameter. The time information and the rate of change can then be used to determine or predict a total change to the parameter. The reliability indicator may be generated responsive to a determination as to whether or not the total change breaches some predetermined threshold (e.g., breach = unreliable, no breach = reliable).

A machine-learning algorithm is any self-training algorithm that processes input data in order to produce or predict output data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The precise content of the training input data entries will depend upon the use for the machine-learning method. The training output data entries may, for instance, be defined by a suitably trained clinician or may be defined in accordance with standard medical guidelines.

By way of example only, where the machine-learning method directly produces the reliability indicator, then the training input data entries correspond to example time information and event information and the training output data entries correspond to indicators of reliability.

As another example, where the machine-learning method produces a time threshold, then the training input data entries correspond to example instances of event information and the training output data entries correspond to time thresholds. As yet another example, where the machine-learning method produces a rate of change, then the training input data entries correspond to example instances of event information and the training output data entries correspond to rates of change.

In some examples, the machine-learning algorithm uses data from previous and/or similar clinical subjects. For instance, data from clinical subjects with similar health conditions and similar size and gender could be used to train the machine-learning algorithm. In some examples, a machine-learning algorithm may even use the previous data from the clinical subject themselves.

Approaches that make use of multiple time thresholds and/or rates of change have been previously described and could be used in embodiments that make use of machine-learning methods (e.g., to produce a time threshold or rate of change for each time segment).

Fig. 4 is a flowchart illustrating a method 400 according to an embodiment.

The method 400 comprises performing the method 200 previously described to generate the reliability indicator for a value of a parameter.

The method 400 also comprises a step 410 of controlling a user interface to provide a visual representation of the reliability indicator. Approaches for providing a visual representation of a reliability indicator will be readily apparent to the skilled person.

In one example, a visual representation of the value for the parameter is modified responsive to the reliability indicator. For instance, an opacity, size, color, thickness, texture, display frequency (e.g., flicker) and/or display pattern of the visual representation of the value may change responsive to the reliability indicator.

The precise modification to the visual representation of the value for the parameter may be dependent upon the nature of the reliability indicator. For instance, if the indicator is a binary indicator, then the modification may be a binary switch between two options (e.g., between two colors). As another example, if the indicator is a numeric indicator, then the change may be more granular. For instance, the opacity or size of the visual representation for the value may decrease with decreasing reliability (as indicated by the numeric indicator). On the other hand, the opposite approach could be chosen, e.g., to make the value stand out more and more, or start flickering, with decreasing reliability to show that the parameter needs to be updated, because the value is becoming decreasingly reliable.

As another example, step 410 may comprise controlling the user interface to provide a dedicated visual representation of the indicator. One example is a pop-up alert responsive to the indicator indicating that the value is unreliable. Another example would be to provide a notification or icon (e.g., an exclamation mark) positioned next to a visual representation of the value for the parameter only when the reliability indicator indicates that the value for the parameter is unreliable.

By way of example, if a reliability indicator indicates that the value of the parameter is unreliable, then a notification might be shown such as a notification might be shown such as: "Please measure the parameter again, because there is a possibility that it will breach a notification threshold" or "Please measure the parameter again, because it is no longer reliable". In some examples, it may even be possible to automatically schedule a new measurement for the parameter (e.g., schedule a new lab measurement).

The method 400 may be iteratively repeated to update the reliability indicator. In some examples, the step 410 is further configured to also display historic or past values for the reliability indicator, so that an individual is able to view the changes in reliability over time.

This approach is particularly advantageous when the reliability indicator indicates that the value of the parameter may (e.g., is likely to), if tested or sampled again, trigger an alarm. In this way, a clinician can be alerted when there is a likelihood above a certain threshold that an alarm would be generated for a value of the alarm at the end point in time, e.g., a current period in time.

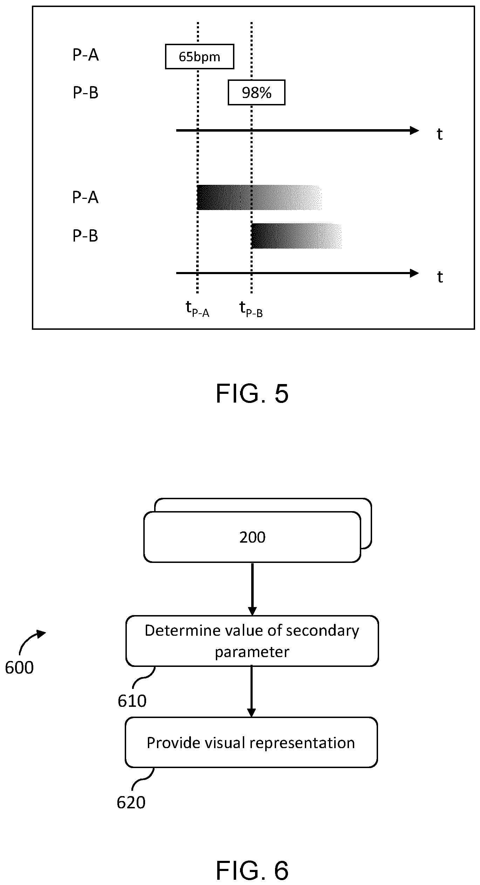

Fig. 5 illustrates one example of a display 500 provided by an example user interface.

The display provides a visual representation 510 of a value for a first parameter P-A (sampled at a time t_{P-A}) and a visual representation 520 of a value for a second parameter P-B (sampled at a time t_{P-B}). By way of example only, the first parameter may be a heartrate (measured in beats per minute (bpm)) and the second parameter may be an SpO2 reading (measured in %).

The display 500 is also configured to provide, for a first parameter P-A and a second parameter P-B, a visual representation of an indicator. Thus, there is a first visual representation 515 of the indicator for the value of the first parameter P-A and a second visual representation 525 of the indicator for the value of the second parameter P-B.

In the illustrated example, each indicator is time-dependent, and thereby identifies a reliability of the (last-recorded value) value of the parameter over time t. Thus, the method 200 of generating an indicator (previously described) is iteratively performed for different points in time, to iteratively determine and update a value for the indicator. Darker portions of each visual representation 510, 520 indicate that the value for the parameter identifies the value being more reliable than light(er) portions of each visual representation.

This approach allows an individual to observe changes in reliability over time. This can allow, for instance, an assessment as to whether a diagnosis or assessment made at a particular point in time was based on reliable data, e.g., and can therefore be trusted.

Other uses for a reliability indicator are hereafter provided. These uses can be performed alongside, or instead of, a step of providing a visual representation of the reliability indicator.

In one example, there may be multiple different mechanisms for generating a value for a particular shared parameter. These different mechanisms can effectively produce different parameters, which share a same goal (and thereby represent a same shared parameter. For instance, for a shared parameter of "blood pressure", one parameter may be a blood pressure monitored using a non-invasive technique whilst another parameter may be a blood pressure monitored using an arterial line. One method may comprise selecting which value represents the shared parameter (e.g., blood pressure) responsive to the reliability indicator of each value. The identified value can, for instance, be displayed in the form of a visual representation and/or further processed, e.g., as exemplified below.

As previously explained, it is common for some parameters of a subject to act as an input parameter (to be processed by an algorithm) to produce a value for another parameter, sometimes labelled a secondary parameter. Put another way, a "secondary parameter" may be produced by combining or processing the value(s) of one or more input parameters. Examples include an early warning score (EWS), hemodynamic stability index (HSI) or a sepsis index.

In such scenarios, a reliability indicator may be a reliability of a value for an input parameter for an algorithm used to produce a secondary parameter. This information can be used to control the production of a secondary parameter.

As one example, the reliability indicator may be used as a weight for the value(s) in an algorithm for producing the processed parameter. In particular, the lower the reliability (as indicated by the reliability indicator) the lower the weighting of the corresponding parameter in an algorithm for producing a secondary parameter.

As another example, there may be a plurality of potential algorithms (e.g., using different input parameter(s)) that could be used to produce a secondary parameter. The reliability indicator(s) of each input parameter for each potential algorithm can be used to select which potential algorithm is used to produce the secondary parameter. In particular, the potential algorithm that uses the input parameter(s) with the greatest (average) reliability may be used to produce the value for the secondary parameter.

As yet another example, production of a value of a secondary parameter may be directly dependent upon the reliability indicator of any/all input parameter(s) processed to produce the value of the secondary parameter. For instance, production of the values for the secondary parameter may be prevented if any reliability indicator for any input parameter is identified as being unreliable.

As another example, a quality measure may be selectively generated for the secondary parameter responsive to the reliability indicator of any/all input parameters (for an algorithm to produce the secondary parameter). In particular, a quality measure may only be generated (using well established quality measure techniques) if the reliability indicator(s) of any/all input parameters indicates that the value for the input parameter is unreliable.

As another example, a visual representation of the value for the secondary parameter may be controlled responsive to the reliability indicator of any/all input parameters (for an algorithm to produce the secondary parameter). This provides an viewer of the value for the secondary parameter with information about the reliability of the value of the secondary parameter. Approaches for controlling the visual representation of a parameter have been previously described, and can be incorporated into this technique.

Fig. 6 is a flowchart that conceptually illustrates a method 600 for determining a value of a secondary parameter.

The method 600 comprises performing a previously described method 200 for determining the reliability indicator(s) of the value(s) of one or more input parameters to be processed to produce the value of the secondary parameter. This may comprise performing multiple instances of the method 200, e.g., if multiple input parameters are to be used.

The method 600 then moves to a step 610 of determining the value of the secondary parameter using the value(s) of the input parameter(s) and their reliability indicator(s). Approaches for performing this step have been previously described.

The method 600 then optionally moves to a step 620 of controlling a user interface to provide a visual representation of the determined value of the secondary parameter. The control of the visual representation may, as previously described, be responsive to the reliability indicator(s) of the input parameter(s).

The skilled person would be readily capable of developing a processing system for carrying out any herein described method. Thus, each step of the flow chart may represent a different action performed by a processing system, and may be performed by a respective module of the processing system.

The processing system may be communicatively connected to an output or user interface.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method (200) for estimating the reliability of a value recorded for a parameter (P, of a clinical subject, the computer-implemented method comprising:
receiving (210) time information that identifies a length of time (T_{P2}) that has elapsed since the value for the parameter was recorded or updated;
receiving (220) event information that identifies the occurrence of any of one or more events (E1, E2, E3) that, when discounting any effect of any other event, are clinically expected to affect the value for the parameter after the value for the parameter was recorded or updated; and
generating (230), by processing the time information and the event information, a reliability indicator that indicates the reliability of the value for the parameter.

2. The computer-implemented method of claim 1, wherein the one or more events comprises at least one interventional event, wherein each interventional event is an event that occurs as a result of an external object or individual interacting with the clinical subject.

3. The computer-implemented method of any of claims 1 to 2, wherein the one or more events comprises at least one environmental event, wherein each environmental event is an event that occurs in a surrounding environment of the clinical subject.

4. The computer-implemented method of any of claims 1 to 3, wherein the one or more events comprises at least one internal event, wherein each internal event is an event that is performed by the clinical subject.

5. The computer-implemented method of claim 4, wherein at least one internal event is a patient-conscious event, being an event that is consciously performed by the clinical subject.

6. The computer-implemented method of claim 4 or 5, wherein at least one internal event is a patient-subconscious event, being an event that is subconsciously performed by the clinical subject.

7. The computer-implemented method of any of claims 1 to 6, further comprising receiving patient history information, wherein
the step of generating the reliability indicator comprises processing the time information, the event information and the patient history information.

8. The computer-implemented method of any of claims 1 to 7, wherein:
each parameter is associated with a notification threshold, being a threshold which, if breached by the value of the parameter, causes a user-perceptible notification to be generated; and
the step of generating the reliability indicator comprises processing the time information, the event information and a determined difference between the value of the parameter and the notification threshold.

9. The computer-implemented method of any of claims 1 to 8, wherein the step of generating the reliability indicator comprises:
looking up a time threshold in a look-up table using at least the event information; and
controlling the reliability indicator to indicate that the value for the parameter is unreliable responsive to the length of time, identified in the time information, breaching the time threshold.

10. The computer-implemented method of any of claims 1 to 8, wherein the step of generating the reliability indicator comprises algorithmically processing the time information and the event information to produce the reliability indicator.

11. The computer-implemented method of any of claims 1 to 10, wherein the value for the parameter is derived from at least one value for one or more physiological parameters of the clinical subject.

12. The computer-implemented method of any of claims 1 to 11, wherein the time information identifies a length of time between a point in time at which the value for the parameter was recorded or updated and a non-current point in time.

13. The computer-implemented method of any of claims 1 to 12, further comprising controlling (620) a user interface to provide a visual representation of the reliability indicator.

14. A computer program product comprising computer program code means which, when executed on a computing device having a processing system, cause the processing system to perform all of the steps of the method according to any of claims 1 to 13.

15. A processing system for determining the reliability of a value recorded for a parameter of a clinical subject, the processing system being configured to:
receive (210) time information that identifies a length of time that has elapsed since the value for the parameter was recorded or updated;
receive (220) event information that identifies the occurrence of any of one or more events that, when discounting any effect of any other event, are clinically expected to affect the value for the parameter after the value for the parameter was recorded or updated; and
generate (230), by processing the time information and the event information, a reliability indicator that indicates the reliability of the value for the parameter.
